# EUROPÄISCHE PATENTANMELDUNG

(11) **EP 0 585 477 A1**
(43) Veröffentlichungstag der Anmeldung: **09.03.1994**
(21) Anmeldenummer: 92114203.0
(22) Anmeldetag: 20.08.1992
(51) Int. Cl.: C07D 249/10, C07D 249/12

(54) **1,2,4-Triazolderivate, Verfahren und Zwischenprodukte zu ihrer Herstellung und ihre Verwendung zur Schädlingsbekämpfung**

(71) Anmelder: BASF Aktiengesellschaft, D-67063 Ludwigshafen (DE)
(72) Erfinder: Kirstgen, Reinhard, Dr., W-6730 Neustadt (DE); Neubauer, Hans-Juergen, Dr., W-4400 Muenster (DE); Otter, Rainer, Dr., W-6947 Laudenbach (DE); Kuenast, Christoph, Dr., W-6701 Otterstadt (DE); Kardorff, Uwe, Dr., W-6800 Mannheim 1 (DE); Harries, Volker, Dr., W-6710 Frankenthal (DE)

(57) **Zusammenfassung**

1,2,4-Triazolderivate der Formel I 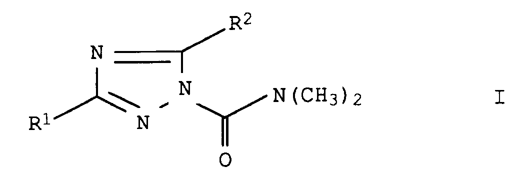
in der R¹ tert.-Butyl oder 1-Methylcyclopropyl bedeutet und R² für Fluor, Chlor, Jod, Thiocyanat und Thiocyanomethylthio steht, und in der R² Brom bedeutet, wenn R¹ für tert.-Butyl steht, Verfahren und Zwischenprodukte zu ihrer Herstellung sowie Mittel und deren Verwendung zur Bekämpfung von Schädlingen.

## Beschreibung

Die vorliegende Erfindung betrifft 1,2,4-Triazolderivate der Formel I 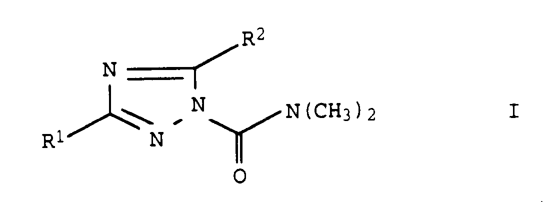
in der R¹ tert.-Butyl oder 1-Methylcyclopropyl bedeutet und R² für Fluor, Chlor, Jod, Thiocyanat und Thiocyanomethylthio steht, und in der R² Brom bedeutet, wenn R¹ für tert.-Butyl steht.

Außerdem betrifft die Erfindung Verfahren und Zwischenprodukte zur Herstellung dieser 1,2,4-Triazolderivate I, Schädlingsbekämpfungsmittel welche derartige 1,2,4-Triazolderivate enthalten sowie Verfahren zur Bekämpfung von Schädlingen mit Hilfe von 1,2,4-Triazolderivaten der Formel IA 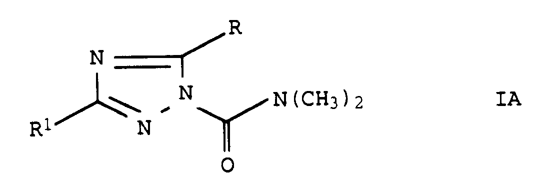
in der R¹ tert.-Butyl oder 1-Methylcyclopropyl bedeutet und R für Halogen, Thiocyanat und Thiocyanomethylthio steht.

Aus der US-A 3 308 131 sind 1,2,4-Triazole der allgemeinen Formeln I'a und I'b bekannt 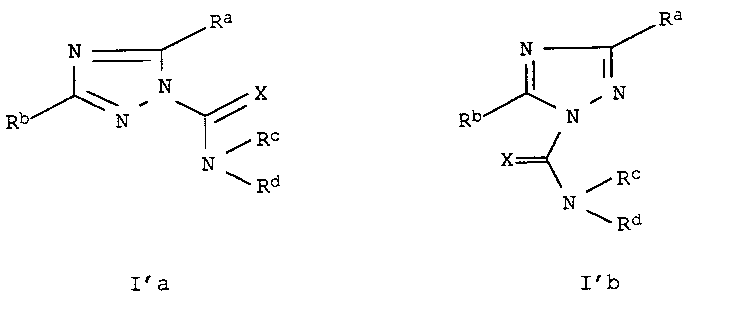
in denen die Reste folgende Bedeutungen haben:
- X: Sauerstoff oder Schwefel;
- R^{c}, R^{d}: aliphatische Reste mit zusammen bis zu 14 C-Atomen und welche einen heterocyclischen Ring mit dem Carbamoylstickstoff bilden können;
- R^{a}, R^{b}: Reste mit zusammen bis zu 14-C-Atomen, die gesättigt sind und aus folgenden Gruppen ausgewählt sein können: Wasserstoff, Halogen, Mercapto, Cyano, Sulfonyl, Kohlenwasserstoff, welcher mit Halogen- oder Nitrogruppe substituiert sein kann, R^{e}O-C(=O)-R^{f}-, R^{e}-S(=O)₂)-, R^{g}-S-, R^{e}-O-R^{f}-;
- R^{e}, R^{f}: Kohlenwasserstoff,
- R^{g}: Kohlenwasserstoff, welcher mit Halogen, Nitro- oder Aminogruppen substituiert sein kann.

Die so beschriebenen Verbindungen sollen insektizide und auch analgetische Wirkungen besitzen. Als Beispiel wird die toxische Wirkung auf Blattläuse an Kapuzinerkresse und Bohnenpflanzen angeführt.

US-A 4 291 043 beschreibt 1-N,N-Dimethylcarbamoyl-3(5)-alkyl-5(3)-alkylthioalkylthio-1,2,4-triazole mit insektizider Wirksamkeit. Die 3(5)-Substituenten umfassen iso-Butyl, sec.-Butyl, tert.-Butyl oder gegebenenfalls methylsubstituiertes Cyclopropyl und ein Rest -S-CH(R')-(CH₂)n-S-R'', worin R' gleich Wasserstoff oder Methyl, R'' gleich (C₁-C₄)-Alkyl und n gleich 0 oder 1 ist.

US-A 3 973 028 und US-A 4 038 387 beschreiben 1-Dimethylcarbamoyl-3-alkyl-1,2,4-triazol-5-yl-(N-substituierte)-sulfonamide mit insektizider Wirksamkeit. Die 3-Alkylsubstituienten umfassen C₃-C₄-sec. und -tert. Alkylgruppen und C₃-C₄-Cycloalkylgruppen.

US-A 4 054 664 beschreibt 1(2)-(N,N-Disubstituierte carbamoyl)-3,5-substituierte-1,2,4-triazole mit insektizider Wirksamkeit. Die 3(5)-Substituenten umfassen iso-Propyl, sec-Butyl, tert.-Butyl und S-R³, wobei R³ gleich Methyl, Ethyl, Propyl, Vinyl, Prop-2-inyl, But-2-enyl oder 2-Halogenalkyl sein kann.

US-A 4 255 435 beschreibt 1(2)-(N,N-Disubstituierte carbamoyl)-3,5-substituierte-1,2,4-triazole mit insektizider und nematizider Wirkung. Die 3(5)-Substituenten umfassen iso-Propyl, sec.-Butyl, tert.-Butyl und S-R⁴, wobei R⁴ gleich Methyl, Ethyl, Propyl, Vinyl, 2-propinyl, 2-Butenyl oder 2-Halogenallyl sein kann.

US-A 4 160 839 beschreibt 1-N,N-Dimethylcarbamoyl-3,5-substituierte-1,2,4-triazole mit insektizider Wirksamkeit. Die 3-Substituenten umfassen tert.-Butyl, Propyl, Cyclopropyl, iso-Propyl oder sec.-Butyl. Die 5-Substituenten umfassen die Gruppe S-R, wobei R gleich 2-Propinyl, Allyl, 2-Bromallyl, 2-Chlorallyl, 2-Methylallyl, 1-Methylallyl oder 2,3,3-Trichlorallyl sein kann.

US-A 4 220 790 und US-A 4 066 774 beschreiben 1-N,N-Dimethylcarbamoyl-3-tert.-butyl-5-methylthiol-1,2,4-triazole mit insektizider Wirksamkeit und ein Verfahren zur Vernichtung von Insekten mit obigem Triazol.

DE-A-3 031 191 beschreibt 1-Dimethylcarbamoyl-3 (oder 5)-benzylthio-5 (oder 3)-alkyl-1,2,4-triazole mit insektizider Wirksamkeit. Die 5 (oder 3)-Substituenten umfassen iso-Propyl, sec.-Butyl, tert.-Butyl oder gegebenenfalls methylsubstituiertes Cyclopropyl.

EP-A-29 047 beschreibt 1-N,N-Dimethylcarbamoyl-3,5-substituierte-1,2,4-triazole mit insektizider Wirksamkeit. Die 3-Substituenten umfassen iso-Propyl, sec.-Butyl, tert.-Butyl oder Cyclopropyl. Die 5-Position trägt einen Rest -S-(CH₂)ₙ-OR, wobei R (C₁-C₃)-Alkyl und n 1 oder 2 bedeutet.

EP-A-213 718 beschreibt 1-N,N-Dimethylcarbamoyl-3,5-substituierte-1,2,4-triazole mit insektizider Wirksamkeit. Die 3-Substituenten umfassen iso-Propyl, sec.-Butyl, tert.-Butyl, tert.-Amyl oder 2-Methylthio-2-propyl. Die 5-Position trägt einen Rest -S-R⁵-X'-R⁶, wobei R⁵ eine gegebenenfalls substituierte, geradkettige C₁-C₆-Alkylidengruppe, X' einen -C(=O)-, -C(=O)-O-, -C(=O)-S-, -(C(=O)-NR³, -SO₂- oder -SO₂-NR³- Rest und R⁶ ein gegebenenfalls substituiertes C₁-C₄-Alkyl, Phenyl-(C₁-C₅)-Alkyl oder Phenyl bedeuten kann.

EP-A-337 815 beschreibt 1-N,N-Dimethylcarbamoyl-3,5-substituierte-1,2,4-triazole mit insektizider Wirksamkeit. Die 3-Substituenten umfassen iso-Propyl, sec.-Butyl, tert.-Butyl, Cyclopropyl, 1-Methylcyclopropyl oder 1-Methylthio-1-methylethyl. Die 5-Position trägt einen Rest -S-R⁷-X, wobei R⁷ eine gegebenenfalls substituierte, geradkettige C₁-C₆-Alkylidengruppe und X eine gegebenenfalls substituierte Di(C₁-C₈)alkoxy-(C₁-C₈)-alkylgruppe oder einen gegebenenfalls substituierten 5-14-gliedrigen Heterocyclus bedeuten kann.

EP-A-338 685 beschreibt 1-N,N-Dimethylcarbamoyl-3,5-substituierte 1,2,4-triazole mit insektizider Wirksamkeit. Die 3-Substituenten umfassen iso-Propyl, sec.-Butyl, tert.-Butyl, Cyclopropyl, 1-Methylcyclopropyl oder 1-Methylthio-1-methylethyl. Die 5-Position trägt einen Rest -S-R⁸-X³, wobei X³ die Bedeutung von -NR^{1x}-C(=O)-R^{x}, -NR^{1x}-C(=S)R^{x} oder -O-C(=O)R^{x} haben kann. R⁸ bedeutet eine gegebenenfalls substituierte C₁-C₁₀-Alkylidengruppe. R^{x} kann Wasserstoff, (C₁-C₈)-Alkoxy, gegebenenfalls substituiertes (C₁-C₈)-Alkoxy, gegebenenfalls substituiertes (C₁-C₈)-Alkyl, mono- oder di-(C₁-C₁₂)Alkylamino, (C₆-C1₄)-Arylamino oder Phenyl bedeuten. R¹x bedeutet Wasserstoff oder gegebenenfalls substituiertes (C₁-C₆)-Alkyl.

Des weiteren sind aus der EP-A 051 565 1-N,N-Dimethylcarbamoyl-3(5)-cyclopropyl-1,2,4-triazole und deren Verwendung zur Bekämpfung von Schädlingen bekannt, deren allgemeine Formel diejenigen Verbindungen I umfaßt, in denen R¹ 1-Methylcyclopropyl und R² Halogen bedeutet.

Außerdem wird in der JP-A 91/068,565 u.a. 1-N,N-Dimethylcarbamoyl-3-tert.-butyl-1,2,4-triazol als Wirkstoff gegen Schädlinge beschrieben.

Aufgabe der vorliegenden Erfindung waren neue 1,2,4-Triazole mit verbesserten Eigenschaften hinsichtlich ihrer Wirksamkeit zur Bekämpfung von Schädlingen.

Demgemäß wurden die eingangs definierten 1,2,4-Triazolderivate der Formel I gefunden.

Außerdem wurden Verfahren und Zwischenprodukte zur Herstellung dieser 1,2,4-Triazolderivate I, Schädlingsbekämpfungsmittel welche derartige 1,2,4-Triazolderivate enthalten sowie Verfahren zur Bekämpfung von Schädlingen mit Hilfe der eingangs definierten 1,2,4-Triazolderivate der Formel IA gefunden.

Man erhält die erfindungsgemäßen 1,2,4-Triazolderivate I, in denen R² für Thiocyanat oder Thiocyanomethylthio steht, dadurch, daß man ein 3,5-disubstituiertes 1H-1,2,4-Triazolderivat IIA in an sich bekannter Weise in einem inerten organischen Lösungsmittel in Gegenwart einer Base mit einem N,N-Dimethylcarbamoylhalogenid III umsetzt. 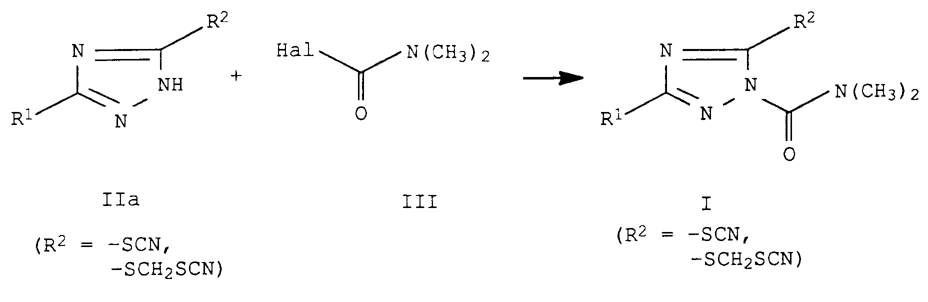
Hal der Formel III steht für ein Halogenatom wie Chlor oder Brom, insbesondere Chlor.

Die Umsetzung wird in der Regel bei Temperaturen von -20°C bis 100°C, vorzugsweise 20°C bis 80°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon und Cyclohexanon sowie Dimethylsulfoxid und Dimethylformamid.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat und Kaliumcarbonat, Natriumcarbonat und Calziumcarbonat sowie Alkalimetallhydrogencarbonate wie Natriumhydrogencarbonat, Alkalimetallalkoholate wie Natriummethanolat und Kalium-tert.butylat, außerdem organische Base, z.B. tertiäre Amine wie Trimethylamin, Trietyhlamin, Di-isopropylethylamin und N-Methylpiperidin, Pyridin, substituierte Pyridine wie Collidin, Lutidin und 4-Dimethylaminopyridin sowie bicyclische Amine in Betracht.

Die Basen werden im allgemeinen in äquimolaren Mengen eingesetzt, sie können aber auch im Überschuß oder gegebenenfalls als Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Die Herstellung der 1,2,4-Triazolderivate der Formel I, in denen R² für Chlor und Jod steht, und in der R² Brom bedeutet, wenn R¹ für tert.-Butyl steht, erfolgt bevorzugt dadurch, daß man ein 3-substituiertes 1H-1,2,4-Triazolderivat IV in an sich bekannter Weise (Z. Chem. 7, 184-185 (1967); Chem. Ber. 100, 2250-2257 (1967); Z. Chem., 9, 300-302 (1969); Khim. Geterotsikl. Soedin 1114-1117 (1969) = Chem. Heterocycl. Comp., 844-847 (1969); Dokl. Akad. Nauk SSSR 220, 101-104 (1975)) in einem inerten Lösungsmittel in Gegenwart einer Base mit einem geeigneten Halogenierungsmittel in ein 5-Halogen-1H-1,2,4-Triazolderivat IIB überführt und dieses anschließend unter den vorstehend beschriebenen Bedingungen mit einem N,N-Dimethylcarbamoylhalogenid III umsetzt. 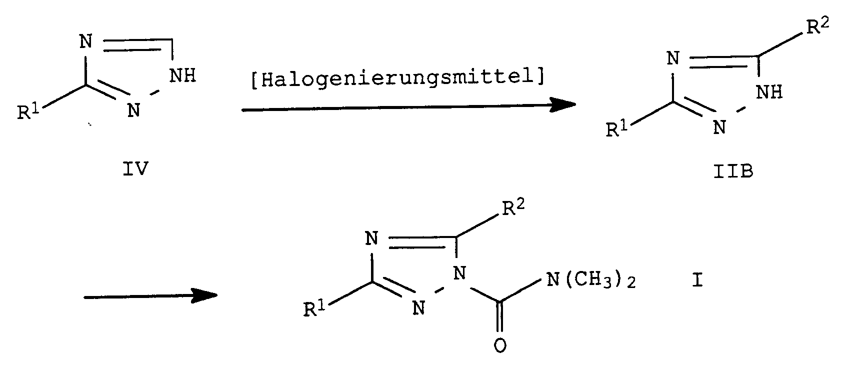
Diese Umsetzung wird üblicherweise bei Temperaturen von -20°C bis -60°C, vorzugsweise -5°C bis 30°C durchgeführt.

Als Halogenierungsmittel eignen sich beispielsweise elementares Chlor oder Brom sowie Jodchlorid oder die entsprechenden Bromverbindungen wie N-Bromsuccinimid.

Geeignete Lösungsmittel sind Wasser, halogenierte Kohlenwasserstoffe wie Methylenchlorid, Chloroform, Tetrachlorkohlenstoff, Dichlorethan und Chlorbenzol, Ether wie Diethylether, Diisopropylether, Dioxan und Tetrahydrofuran, besonders bevorzugt Wasser, Chloroform, Tetrachlorkohlenstoff, Tetrahydrofuran und Diethylether.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid, Calziumhydroxid, Alkalimetall- und Erdalkalimetalloxide wie Lithiumoxid, Natriumoxid, Calziumoxid und Magnesiumoxid, Alkalimetall- und und Erdalkalimetallcarbonate wie Lithiumcarbonat, Natriumcarbonat, Kaliumcarbonat und Calziumcarbonat sowie Alkalimetallhydrogencarbonate wie Natriumhydrogencarbonat und Kaliumhydrogencarbonat, sowie Alkalimetallhydride wie Natriumhydrid in Betracht.

Besonders bevorzugt werden Natriumhydroxid, Natriumcarbonat, Kaliumcarbonat, Natriumhydrogencarbonat und Kaliumhydrogencarbonat.

Die Basen werden im allgemeinen im Überschuß oder gegebenenfalls als Lösungsmittel verwendet werden.

Das Halogenierungsmittel wird im allgemeinen in äquimolaren Mengen eingesetzt. Es kann für die Ausbeute vorteilhaft sein, das Halogenierungsmittel in einem Überschuß bezogen auf 1,2,4-Triazolderivat einzusetzen.

Die z.T. bei den vorstehend beschriebenen Halogenierungen in einem ersten Schritt entstehenden 1-Halogen-1,2,4-triazolderivate können z.B. mit überschüssigem Natriumhydrogencarbonat zu 1-H-1,2,4-Triazolderivaten IIB reduziert werden (Z. Chem. 7, 184-185 (1967)) oder durch Erhitzen in Halogenkohlenwasserstoffen wie beschrieben, in Wasser oder in Alkoholen wie tert. Butanol zu den gewünschten Derivaten IIB (J. Prakt. Chem. 314, 923-935 (1972)) umgelagert werden.

Bei den oben beschriebenen Herstellungsverfahren können z.T. die stellungsisomeren Verbindungen als Gemische entstehen. Die Isomere mit der erfindungsgemäßen Konfiguration können in allen Fällen durch chromatographische Trennung erhalten werden.

Außerdem können die 1,2,4-Triazolderivate der Formel I, in denen R² für Fluor, Chlor und Jod steht, und in denen R² Brom bedeutet, wenn R¹ für tert.-Butyl steht, bzw. deren Vorstufen der Formel IIB auch in Analogie zu anderen literaturbekannten Methoden, z.B. durch Diazotierung von 5-Amino-1H-1,2,4-triazolen in Gegenwart von geeigneten Halogeniden (Chem. Ber., 100, 2250-2257 (1967); Methody Poluch. Khim. Reaktiv Prop., 75-76 (1971)) hergestellt werden.

Die als Edukte verwendeten 3,5-disubstituierten 1 H-1,2,4-Triazolderivate II 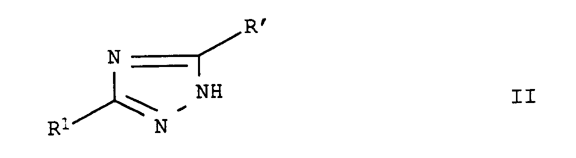
in denen R¹ tert.-Butyl oder 1-Methylcyclopropyl bedeutet und R' für Halogen, Thiocyanat und Thiocyanomethylthio steht sind neu.

Diejenigen Derivate, in denen R' für Halogenatome steht, können nach den vorstehend geschilderten Verfahren hergestellt werden.

Derivate, in denen R' für Thiocyanat und Thiocyanomethylthio steht, können in Analogie zu bekannten Methoden aus den Verbindungen V erhalten werden: 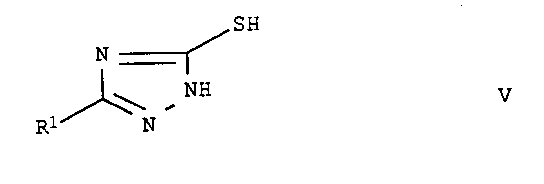
a) Umsetzung mit Cyanhalogeniden wie Chlorcyan und Bromcyan, ggf. in Gegenwart einer Base zu Thiocyanaten (Potts et al., J. Org. Chem., 31, 3528 (1966); Koeppe et al., J. Am. Chem. Soc., 75, 4655 (1953))
b) Umsetzung mit Methylendirhodanid zu Thiocyanomethylthio-Derivaten (EP-A 317 912)
Die vorliegende Erfindung umfaßt die folgenden Verbindungen:

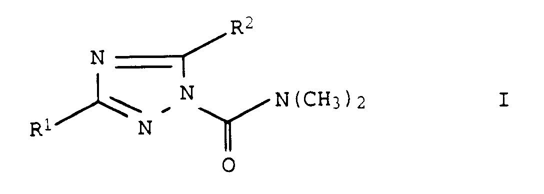

| Nr. | R¹ | R² |
|---|---|---|
| 01 | C(CH₃)₃ | F |
| 02 | C(CH₃)₃ | Cl |
| 03 | C(CH₃)₃ | J |
| 04 | C(CH₃)₃ | SCN |
| 05 | C(CH₃)₃ | SCH₂SCN |
| 06 | C(CH₃)₃ | Br |
| 07 | 1-CH₃-cyclopropyl | F |
| 08 | 1-CH₃-cyclopropyl | Cl |
| 09 | 1-CH₃-cyclopropyl | J |
| 10 | 1-CH₃-cyclopropyl | SCN |
| 11 | 1-CH₃-cyclopropyl | SCH₂SCN |

Im Hinblick auf ihre biologische Wirksamkeit sind insbesondere die Verbindungen Nr. 04 (1-N,N-Dimethylcarbamoyl-3-tert.-butyl- 5-thiocyano-1H-1,2,4-triazol), Nr. 05 (1-N,N-Dimethylcarbamoyl-3- tert.-butyl-5-thiocyanomethylthio-1H-1,2,4-triazol) und Nr. 06 (1-N,N-Dimethylcarbamoyl-3-tert.-butyl-5-brom-1H-1,2,4-triazol) bevorzugt.

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femurrubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnapthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoryliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

### Beispiele für Formulierungen sind:

I. 5 Gew.-Teile der Verbindung aus Beispiel 04 werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile der Verbindung aus Beispiel 05 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile der Verbindung aus Beispiel 04 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile der Verbindung aus Beispiel 05 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile der Verbindung aus Beispiel 04 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile der Verbindung aus Beispiel 04 mit 10 Gew.-Teilen N-Methyl-a-pyrrolidon und erhält eine Lösung, die zur Anwendung in Form kleinster Tropfen geeignet ist (Wirkstoffgehalt 90 Gew.%).
VII. 20 Gew.-Teile der Verbindung aus Beispiel 04 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile des Wirkstoffs aus Beispiel 05 werden mit 3 Gew.-Teilen des Natriumsalzes der Diisobutylnaphthalin-a-sulfonsäure, 17 Gew.-Teilen des Natriumsalzes einer Ligninsulfonsäure aus einer Sulfit-Anlauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniunitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften können unter entsprechender Abwandlung der Ausgangsverbindungen zur Gewinnung weiterer Verbindungen I genutzt werden. Die auf diesen Wegen synthetisierten Beispielverbindungen sind in der anschließenden Tabelle mit physikalischen Daten aufgeführt.

### Vorschrift 1

### Darstellung von 3-t-butyl-5-thio-1H-1,2,4-triazol

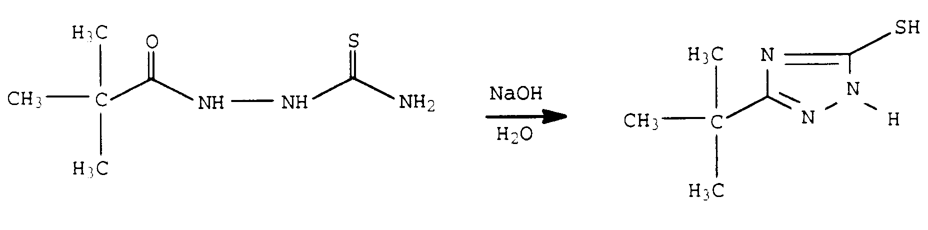

Zu einer Lösung von 125 g Natriumhydroxid in 600 ml Wasser werden bei Raumtemperatur 262,5 g N-(tert.Butylcarbonyl)thiosemicarbazid gegeben. Der Ansatz wird dann auf 90°C erhitzt und nachdem alles in Lösung gegangen ist, noch eine Stunde bei dieser Temperatur gerührt. Nach Abkühlen auf 5 bis 10°C wird mit konz. Salzsäure auf pH 2 bis 3 eingestellt. Hierbei fällt das Produkt aus, wird abfiltriert und mit Wasser neutral gewaschen. Zur weiteren Reinigung wird das Rohprodukt in Trichlormethan gelöst, die Lösung mit Natriumsulfat getrocknet, abfiltriert und schließlich das 3-t-Butyl-5-thio-1H-1,2,4-triazol durch Zugabe von Cyclohexan wieder ausgefällt. Man erhält 124 g 3-t-Butyl-5-thio-1H-1,2,4-triazol mit Schmelzpunkt 198 bis 203°C.

### Vorschrift 2

### Darstellung von 3-t-Butyl-5-thiocyanomethylthio-1H-1,2,4-triazol

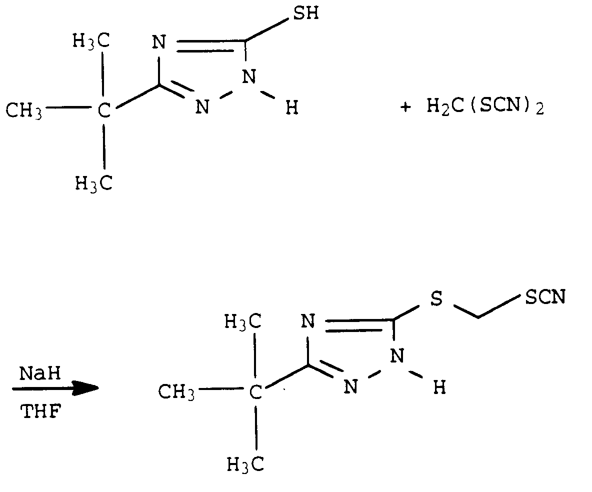

In einer 250 ml Rührapparatur werden unter Stickstoff 7,8 g 3-t-Butyl-5-thio-1H-1,2,4-triazol in 30 ml wasserfreiem Tetrahydrofuran vorgelegt und portionsweise mit 1,22 g Natriumhydrid versetzt. Nach beendeter Wasserstoffentwicklung wird die Suspension zu 6,5 g Methylendirhodanid in 30 ml wasserfreiem Tetrahydrofuran getropft. Nach leicht exothermer Reaktion wird weitere 3 Stunden bei Raumtemperatur gerührt. Zur Aufarbeitung gießt man den Ansatz in Wasser/Methyl-tert-butylether (1:1), trennt die wäßrige Phase ab, extrahiert mehrmals mit Methyl-tert.-butylether und trocknet die vereinigten organischen Phasen mit Natriumsulfat. Nach Abziehen des Lösungsmittels verbleiben 10,5 g 3-t-Butyl-5-thiocyanomethylthio-1H-1,2,4-triazol als Öl.

IR[cm-1]: 2976, 2156, 1552, 1414, 1366, 1261, 1209, 1033

### Vorschrift 3

### Darstellung von 3-t-Butyl-5-thiocyano-1H-1,3,4-triazol

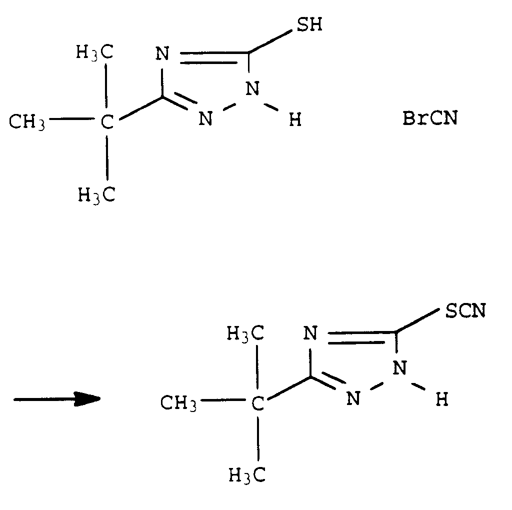

4,7 g fein gepulvertes 3-t-Butyl-5-thio-1H-1,2,4-triazol und 9,5 g Bromcyan werden in einem N₂-gespültem 100 ml Kolben intensiv gemischt und anschließend auf 50 bis 550°C aufgewärmt. Dabei schmilzt das Bromcyan auf. Nach 30 min. wird der Ansatz eine Stunde mit Stickstoff gespült. Der verbleibende hellgelbe Feststoff wird gepulvert, nochmals mit 9,5 g Bromcyan versetzt und wieder auf ca. 50°C erwärmt. Nach 30 min wird der pastöse Ansatz auf 60°C erwärmt und überschüssiges Bromcyan durch mehrstündiges Spülen mit Stickstoff vertrieben. Zur Aufarbeitung wird der verbleibende Feststoff im Eiswasser eingerührt und die Lösung mit NaHCO auf pH 7 bis 8 eingestellt. Man extrahiert mehrmals mit Dichlormethan, wäscht die vereinigten organischen Phasen mit gesättigter NaCl-Lösung und trocknet mit Natriumsulfat. Nach Abziehen des Lösungsmittels verbleiben 3,6 g 3-t-Butyl-5-thiocyano-1H-1,2,4-triazol mit Schmelzpunkt 138 bis 140°C.

### Vorschrift 4

### Darstellung von 3-t-Butyl-1H-1,2,4-triazol

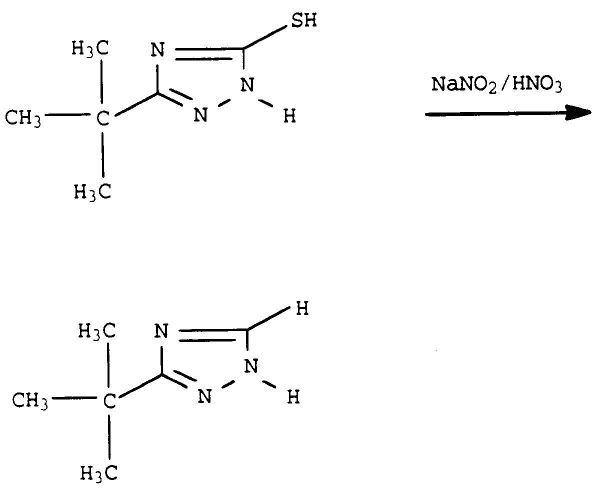

Zu einer Lösung von 34,5 g Natriumnitrit und 300 ml konzentrierter Salpetersäure in 2 l Wasser werden portionsweise 234 g 3-t-Butyl-5-thio-1H-1,2,4-triazol gegeben. Bei der stark exothermen Reaktion soll die Temperatur nicht über 45°C steigen. Nach vollständiger Zugabe wird über Nacht bei Raumtemperatur (20°C) gerührt. Unter Eiskühlung wird dann vorsichtig mit 2N Natronlauge neutralisiert und anschließend fast zur Trockene eingeengt. Der Rückstand wird in Ethanol aufgenommen und kurz bis zum Rückfluß erhitzt. Der nach dem Abkühlen verbleibende Feststoff wird abgesaugt und mit kaltem Ethanol gewaschen. Die vereinigten Filtrate werden zur Trockene eingeengt. Man erhält 148,7 g kristallines 3-t-Butyl-1H-1,2,4-triazol mit Schmelzpunkt 183 bis 185°C.

### Vorschrift 5

### Darstellung von 3-t-Butyl-5-brom-1H-1,2,4-triazol

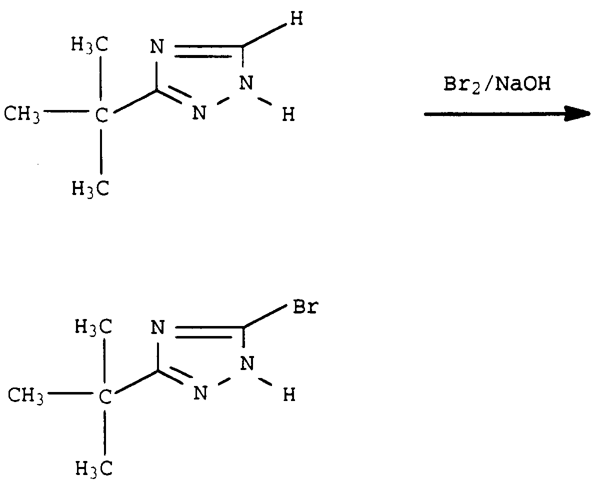

Zu einer Suspension von 148 g 3-t-Butyl-1H-1,2,4-triazol in 2 l Wasser gibt man 120 ml 50 %ige Natronlauge. Zu der gelben Lösung tropft man 190,4 g Brom und rührt 2 Stunden bei Raumtemperatur nach. Der Ansatz wird mit verd. Salzsäure auf pH-Wert 3 bis 4 eingestellt und mehrmals mit Ethylacetat extrahiert. Die vereinigten organischen Phasen werden eingeengt. Das Rohprodukt kann durch Umkristallisieren aus Ethylacetat/n-Hexan gereinigt werden. Man erhält 184 g 3-t-Butyl-5-brom-1H-1,2,4-triazol mit Schmelzpunkt 179 bis 181°C.

### Beispiel 1

### Darstellung von 1-N,N-Dimethylcarbamoyl-3-t-butyl-5-thiocyanomethylthio-1H-1,2,4-triazol

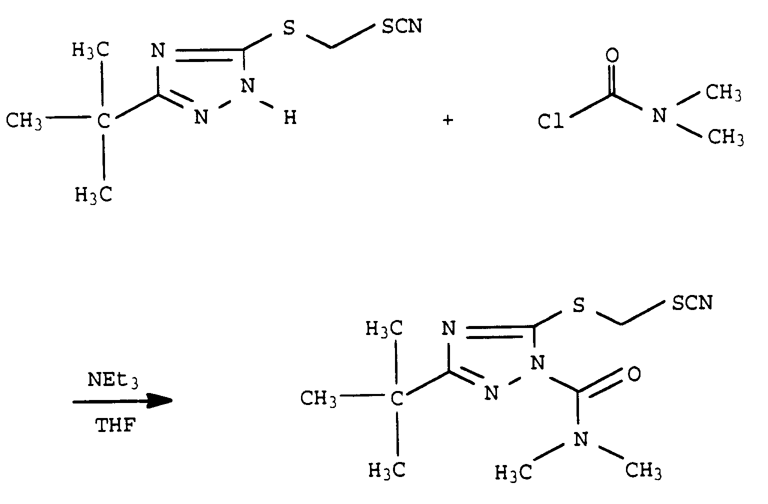

Zu einer Lösung von 5,7 g 3-t-Butyl-5-thiocyanomethylthio-1H-1,2,4-triazol, 2,7 g Dimethylcarbamoylchlorid und 0,6 g 4-Dimethylaminopyridin in 75 ml wasserfreiem Tetrahydrofuran werden bei Raumtemperatur innerhalb von 30 min 5,0 g Triethylamin zugetropft. Danach wird 4 h am Rückfluß gekocht und über Nacht bei Raumtemperatur gerührt. Zur Aufarbeitung wird der Ansatz eingeengt und mit 100 ml Ethylacetat/Wasser aufgenommen. Die abgetrennte organische Phase wird mehrmals mit Wasser gewaschen, mit Natriumsulfat getrocknet und eingeengt. Nach vollständiger Entfernung des Lösungsmittels erhält man 55 g 1-N,N-Dimethylcarbamoyl-3-t-butyl-5-thiocyanomethylthio-1H-1,2,4-trizol als wachsartige Substanz.

IR [cm-1]: 2967, 2150, 1693, 1512, 1393, 1320, 1058

### Beispiel 2

### Darstellung von 1-N,N-dimethylcarbamoyl-3-t-butyl-5-thiocyano-1H-1,2,4-triazol

Aus 3,5 g 3-t-Butyl-5-thiocyano-1H-1,2,4-triazol erhält man analog dem Verfahren im Herstellungsbeispiel 1 nach Verreiben mit n-Pentan 3,1 g 1-N,N-Dimethylcarbamoyl-3-t-butyl-5-thiocyano-1H-1,2,4-triazol mit Schmelzpunkt 92 bis 94°C.

### Beispiel 3

### Darstellung von 1-N,N-Dimethylcarbamoyl- 3-t-butyl-1H-1,2,4-triazol

Aus 4,0 g 3-t-Butyl-1H-1,2,4-triazol erhält man analog dem Verfahren in Herstellungsbeispiel 1 nach säulenchromatographischer Reinigung 1,9 g 1-N,N-Dimethylcarbamoyl-3-t-butyl-1H-1,2,4-triazol mit Schmelzpunkt 52 bis 54°C.

**Tabelle**

| Nr. | R¹ | R² | physik. Daten |
|---|---|---|---|
| 04 | C(CH₃)₃ | SCN | IR: 2967, 2150, 1693, 1512, 1393, 1320, 1058 cm⁻¹ |
| 05 | C(CH₃)₃ | SCH₂SCN | Fp: 92-94°C |
| 06 | C(CH₃)₃ | Br | Fp: 52-54°C |

### Anwendungsbeispiele

Die insektizide Wirkung der Verbindungen der allgemeinen Formel I ließ sich durch folgende versuche zeigen:

Die Wirkstoffe wurden
a) als 0,1 %ige Lösung in Aceton oder
b) als 10 %ige Emulsion in einem Gemisch aus 70 Gew.% Cyclohexanon, 20 Gew.% Nekanil® LN (Lutensol® AP6, Netzmittel mit Emulgier- und Dispergierwirkung auf der Basis ethoxylierter Alkylphenole) und 10 Gew.% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80-100 %ige Hemmung bzw. Mortalität hervorriefen (Wirkschwelle bzw. Minimalkonzentration).

Die folgenden Versuche wurden durchgeführt:
A.1 Aphis fabae (Schwarze Laus), Kontaktwirkung
   Stark befallene Buschbohnen (Vicia faba) wurden mit der wässrigen Wirkstoffaufbereitung behandelt.
   Nach 24 h wurde die Mortalitätsrate bestimmt.
A.2 Aphis fabae (Schwarze Laus), Systemwirkung
   Stark befallene Buschbohnen (Vicia faba) wurden mit der wässrigen Wirkstoffaufbereitung gegossen, wobei der Wirkstoff im wesentlichen auf den Boden appliziert wird. Nach 24 h wurde die Mortalitätsrate bestimmt.
A.3 Blatella germanica (Deutsche Schabe), Kontaktwirkung
   Der Boden eines Versuchsgefäßes wurde mit der acetonischen Lösung des Wirkstoffs benetzt und nach dem Abdampfen des Lösungsmittels mit 10 adulte Schaben besetzt.
   Nach 48 h wurde die Mortalitätsrate bestimmt.
A.4 Caenorhabditis elegans (Freilebende Nematoden), Kontaktwirkung
   Der Boden eines Versuchsgefäßes wurde mit der acetonischen Lösung des Wirkstoffs benetzt und nach dem Abdampfen des Lösungsmittels mit E.-Coli-Bakteriensuspension als Nährmedium bedeckt und mit 50 µl Nematodensuspension infiziert.
   Nach 48 h wurde die Mortalitätsrate bestimmt.
A.5 Epilachna varivestis (Mex. Bohnenkäfer), Kontaktwirkung (Larven)
   Die Innenwand eines Versuchsgefäßes wurde mit der acetonischen Wirkstoffaufbereitung behandelt. Nach dem Abdampfen des Lösungsmittels wurden 5 Larven (ca. 3-4 mm) in das Gefäß gebracht.
   Nach 24 h wurde die Mortalitätsrate bestimmt.
A.6 Leptinotarsa decemlineata (Kartoffelkäfer), Kontakt-/Fraßwirkung (Larven)
   Frische Kartoffelblätter wurden mit der wäßrigen Wirkstoffaufbereitung behandelt und nach dem Trocknen mit 10 Larven belegt.
   Nach 48 h wurde die Mortalität beurteilt.
A.7 Leptinotarsa decemlineata (Kartoffelkäfer), Kontakt-/Fraßwirkung (Adulte)
   Frische Kartoffelblätter wurden mit der wäßrigen Wirkstoffaufbereitung behandelt und nach dem Trocknen mit 10 adulten Käfern belegt.
   Nach 24h und 48 h wurde die Mortalität und die Fraßminderung beurteilt.
A.8 Megoura viciae (Wickenblattlaus), Systemwirkung
   Bohnenpflanzen des 4. Blattstadiums wurden mit der wäßrigen Wirkstoffaufbereitung behandelt und anschließend mit ca. 30-40 Blattläusen belegt.
   Nach 48 h wurde die Mortalität beurteilt.
A.9 Musca domestica (Stubenfliege), Kontaktwirkung Der Boden eines Versuchsgefäßes wurde mit der acetonischen Lösung des Wirkstoffs benetzt und nach dem Abdampfen des Lösungsmittels mit 10 Fliegen besetzt.
   Nach 4 h wurde die Mortalitätsrate bestimmt.
A.10 Nephotettix cincticeps (Reiszikade), Kontaktwirkung (Spritzversuch)
   Ca. 8 cm große Reispflanzen wurden mit der wäßrigen Wirkstoffaufbereitung behandelt und nach dem Abtrocknen mit 10 adulten Zikaden belegt.
   Nach 48 h wurde die Mortalität beurteilt.
A.11 Oncopeltus sp. (Baumwollwanze), Kontaktwirkung
   Der Boden eines Versuchsgefäßes wurde mit der acetonischen Wirkstoffaufbereitung behandelt und nach dem Verdunsten des Lösungsmittels mit 5 adulten Wanzen belegt.
   Nach 24 h wurde die Mortalitätsrate bestimmt.
A.12 Ornithodorus moubata (Zecke), Kontaktwirkung
   Je 5 Zecken (Durchmesser 1,5 - 2 mm; nach einer Blutmahlzeit) wurden für ca. 5 sec. in die wässrige Wirkstoffaufbereitung getaucht.
   Nach 48 h wurde die Mortalitätsrate bestimmt.
A.13 Tribolium sp. (Reismehlkäfer), Kontaktwirkung
   Der Boden eines Versuchsgefäßes wurde mit der acetonischen Wirkstoffaufbereitung behandelt und nach dem Verdampfen des Lösungsmittels mit 10 adulten Käfern belegt.
   Nach 24 h wurde die Mortalität bestimmt.

Der Vergleich der biologischen Aktivität der erfindungsgemäßen Verbindungen und der bekannten Wirkstoffe A (1-N,N-Dimethyl-carbamoyl-3-tert.-butyl-1H-1,2,4-triazol; Beispiel Nr. 1 aus JP-A 91/068,565) und B (1-N,N-Dimethylcarbamoyl-3-tert.-butyl-5-methylthio-1H-1,2,4-triazol; Beispiel Spalte 2, Zeile 30, aus US-A 4,054,664) ergab die in den folgenden Tabellen A, B und C zusammengestellten Ergebnisse.

**Tabelle A**

| Test | Schädling-Wirkungsart | Verbindung Nr. 06 | | Verbindung A | |
|---|---|---|---|---|---|
| | | ppm | % | ppm | % |
| A.4 | Caenorhabditis elegans (Adulte) - Kontakt/Fraß | 10 | 100 | 100 | 100 |
| A.12 | Ornithodorus moubata (Larven) - Kontakt/Fraß | 100 | 100 | 200 | 90 |

**Tabelle B**

| Test | Schädling-Wirkungsart | Verbindung Nr. 06 | | | | |
|---|---|---|---|---|---|---|
| | | 04 | 05 | 06 | A | B |
| | | [ppm/%] | | | | |
| A.1 | Aphis fabae - Kontakt | nt | nt | 40 | 40 | 40 |
| | | -- | -- | 100 | 80 | 80 |
| A.2 | Aphis fabae - System | nt | 400 | 20 | 20 | 4 |
| | | -- | 100 | 80 | 80 | 80 |
| A.8 | Megoura viciae - System | nt | nt | 20 | 40 | 20 |
| | | -- | -- | 80 | 80 | 80 |
| A.10 | Nephotettix cincticeps - Kontakt | 200 | 400 | 40 | 40 | 4 |
| | | 80 | 80 | 100 | 100 | 100 |
| nt = nicht getestet | | | | | | |

**Tabelle C**

| Test | Schädling-Wirkungsart | Verbindung Nr. 06 | | Verbindung A | |
|---|---|---|---|---|---|
| | | ppm | % | ppm | % |
| A.3 | Blatella germanica - Kontakt | 40 | 100 | 400 | 100 |
| A.5 | Epilachna varivestis (Larven) - Kontakt | 1 | 100 | 40 | 100 |
| A.6 | Leptinotarsa decemlineata (Larven) - Kontakt/Fraß | 40 | 100 | 100 | 100 |
| A.7 | Leptinotarsa decemlineata (Adulte) - Kontakt/Fraß | 100 | 100 | 400 | 100 |
| A.8 | Megoura viviae - System | 40 | 100 | 100 | 80 |
| A.9 | Musca domestica - Kontakt | 100 | 100 | 1000 | 100 |
| A.10 | Nephotettix cincticeps - System | 10 | 100 | 10 | 80 |
| A.11 | Oncopeltus sp. (Adulte) - Kontakt | 2 | 100 | 20 | 100 |
| A.13 | Tribolium sp. (Adulte) - Kontakt | 20 | 100 | 100 | 100 |

## Patentansprüche

1. 1,2,4-Triazolderivate der Formel I 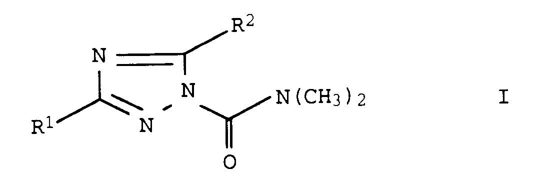 in der R¹ tert.-Butyl oder 1-Methylcyclopropyl bedeutet und R² für Fluor, Chlor, Jod, Thiocyanat und Thiocyanomethylthio steht, und in der R² Brom bedeutet, wenn R¹ für tert.-Butyl steht.

2. Schädlingsbekämpfungsmittel, enthaltend eine wirksame Menge eines 1,2,4-Triazolderivats der Formel I gemäß Anspruch 1 sowie inerte Trägerstoffe.

3. Verfahren zur Bekämpfung von Schädlingen, dadurch gekennzeichnet, daß man die Schädlinge und/oder die vor Schädlingsbefall zu schützenden Pflanzen, Saatgüter, Materialien oder den Erdboden mit einer wirksamen Menge eines 1,2,4-Triazolderivats der Formel IA 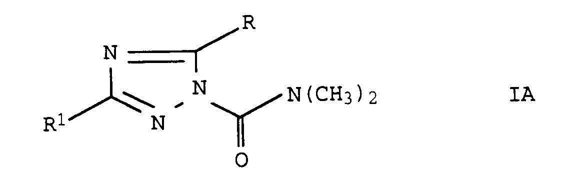 in der R¹ tert.-Butyl oder 1-Methylcyclopropyl bedeutet und R für Halogen, Thiocyanat und Thiocyanomethylthio steht, behandelt.

4. Verfahren zur Herstellung eines 1,2,4-Triazolderivats der Formel I gemäß Anspruch 1, in der R² für Thiocyanat oder Thiocyanomethylthio steht, dadurch gekennzeichnet, daß man ein 3,5- disubstituiertes 1H-1,2,4-Triazolderivat der Formel IIA 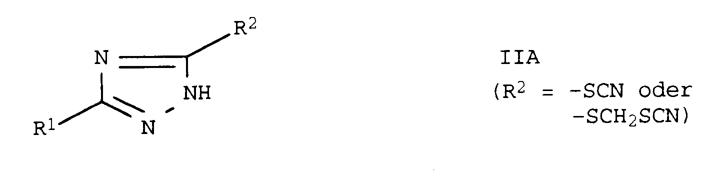 in an sich bekannter Weise in einem inerten organischen Lösungsmittel in Gegenwart einer Base mit einem N,N-Dimethylcarbamoylhalogenid der Formel III 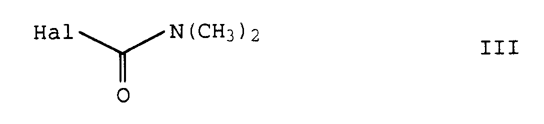 in der Hal für ein Halogenatom steht, umsetzt.

5. Verfahren zur Herstellung eines 1,2,4-Triazolderivats der Formel I gemäß Anspruch 1, in der R² für Fluor, Chlor und Jod, steht, und in der R² Brom bedeutet und R¹ für tert.-Butyl steht, dadurch gekennzeichnet, daß man ein 3-substituiertes 1 H-1,2,4-Triazolderivat der Formel IV 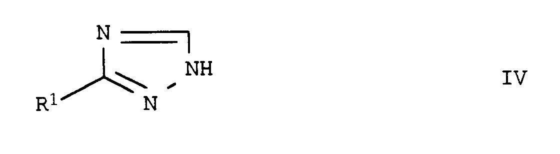 in an sich bekannter Weise in einem inerten Lösungsmittel in Gegenwart einer Base mit einem geeigneten Halogenierungsmittel in ein 5-Halogen-1H-1,2,4-Triazolderivat der Formel IIB 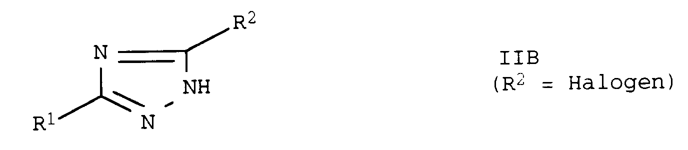 überführt und dieses anschließend in an sich bekannter Weise in einem inerten organischen Lösungsmittel in Gegenwart einer Base mit einem N,N-Dimethylcarbamoylhalogenid der Formel III gemäß Anspruch 4, umsetzt.

6. 3,5-Disubstituierte 1H-1,2,4-Triazolderivate der Formel II 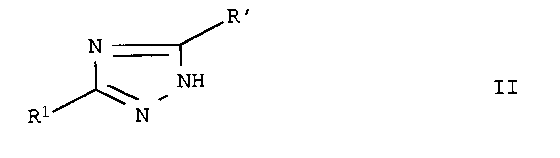 in der R¹ tert.-Butyl oder 1-Methylcyclopropyl bedeutet und R' für Halogen, Thiocyanat und Thiocyanomethylthio steht.

7. 1-N,N-Dimethylcarbamoyl-3-tert.-butyl-5-thiocyano-1H-1,2,4-triazol.

8. 1-N,N-Dimethylcarbamoyl-3-tert.-butyl-5-thiocyanomethylthio-1H-1,2,4-triazol.

9. 1-N,N-Dimethylcarbamoyl-3-tert.-butyl-5-brom-1H-1,2,4-triazol.
